# EUROPÄISCHE PATENTSCHRIFT

(11) **EP 1 418 165 B1**
(45) Veröffentlichungstag und Bekanntmachung des Hinweises auf die Patenterteilung: **09.09.2009**
(21) Anmeldenummer: 03024748.0
(22) Anmeldetag: 29.10.2003
(51) Int. Cl.: C07C 29/141

(54) **Verfahren zur Gewinnung von aliphatischen C3-C10-Alkoholen aus Hochsiedern**
Process for producing aliphatic C3-C10 alcohols from high boiling compounds
Procédé de préparation d'alcools en C3-C10 à partir de composés à point d'ébullition élevé

(30) Priorität: 09.11.2002 DE 10252173
(43) Veröffentlichungstag der Anmeldung: 12.05.2004
(73) Patentinhaber: OXEA GmbH, 46147 Oberhausen (DE)
(72) Erfinder: Zgorzelski, Wolfgang, 46149 Oberhausen (DE); Gick, Wilhelm, Dr., 47199 Duisburg (DE)

(56) Entgegenhaltungen:
- EP-A- 0 216 151
- US-A- 5 227 544

## Beschreibung

Die vorliegende Erfindung betrifft ein Verfahren zur Gewinnung von aliphatischen C₃-C₁₀-Alkoholen aus Hochsiedern durch thermische Behandlung in Gegenwart von Alkalimetallverbindungen und nachfolgender Hydrierung der flüchtigen Produkte.

Aliphatische C₃-C₁₀-Alkohole, wie n-Butanol und insbesondere 2-Ethylhexanol besitzen eine hohe wirtschaftliche Bedeutung. Die Herstellung dieser Alkohole erfolgt bevorzugt durch die Hydroformylierung von Olefinen mit nachfolgender Hydrierung der intermediär gebildeten Aldehyde. Ein wirtschaftlich bedeutendes Beispiel ist die Hydroformylierung von Propylen zu n/i-Butyraldehyd und die anschließende Hydrierung zu n/i-Butanol. Einen weiteren Zugang zu aliphatischen Alkoholen ermöglicht die Aldolisierung von geradkettigen aliphatischen Aldehyden zu den entsprechenden ungesättigten Aldehyden mit nachfolgender Hydrierung. Ein wichtiges Beispiel für diesen Prozess ist die Herstellung von 2-Ethylhexanol ausgehend von n-Butyraldehyd über die Zwischenstufe 2-Ethylhexenal. Zusammenfassende Darstellungen finden sich z.B. in Ullmann's Encyclopedia of Industrial Chemistry: "Alcohols, Aliphatic" (Vol. A1), "2-Ethylhexanol" (Vol. 10) und "Butanols" (Vol. A4).

Außer als Lösungsmittel wird n-Butanol vor allem auf dem Farb- und Lacksektor und zur Herstellung von Carbonsäureestern, insbesondere n-Butylacrylat und Di-n-butylphthalat (DBP) verwendet. 2-Ethylhexanol wird vornehmlich als Alkoholkomponente zur Herstellung von Di-2-ethylhexylphthalat (DEHP) und 2-Ethylhexylacrylat benötigt.

Für diese Anwendungsbereiche ist der Einsatz hochreiner Alkohole gewünscht und in manchen Fällen, wie zum Beispiel bei der Herstellung von Acrylsäureestern, sogar zwingend notwendig. Bei der technischen Herstellung der Alkohole erfolgt die Reinigung durchwegs durch mehrstufige fraktionierte Destillation. Die Alkohole werden hierbei über einen Zeitraum von mehreren Stunden einer thermischen Belastung ausgesetzt, wobei in der Regel Sumpftemperaturen von 150 bis 200°C angewandt werden. Als Folge hiervon kommt es bei der Destillation von aliphatischen C₃-C₁₀-Alkoholen zur Bildung von Hochsiedern, die mit dem Sumpfabzug aus der Alkoholdestillationskolonne ausgeschleust werden. Weiterhin fallen während des Herstellprozesses von aliphatischen C₃-C₁₀-Alkoholen Hochsieder an. Insbesondere beobachtet man bei der der 2-Ethylhexanol-Herstellung vorgeschalteten Aldolisierung von n-Butyraldehyd zu 2-Ethylhexenal und der anschließenden 2-Ethylhexenal-Destillation die Bildung von Hochsiedern.

Die Hochsiederbildung bei der Herstellung von aliphatischen C₃-C₁₀-Alkoholen bedeutet einen wirtschaftlichen Nachteil, weil durch die Hochsiederbildung eine erhebliche Menge an Wertprodukt verloren geht und somit die Alkoholausbeute gemindert wird. Zudem ist die Entsorgung des Hochsiederanfalls aufwendig und teuer. Es besteht daher ein Bedarf an einem Verfahren, den Hochsiederanfall während der Herstellung und destillativen Reinigung der aliphatischen C₃-C₁₀-Alkohole zu reduzieren.

Aus dem Stand der Technik ist bekannt, Alkohole unter Zusatz von basischen Verbindungen zu destillieren. Nach dem aus EP-B1-0 869 936 bekannten Verfahren bewirkt der Zusatz von 10 bis 1000 ppm Alkalihydroxid während der Alkoholdestillation eine Verbesserung der CO-Zahl des gewonnenen Alkohols. Die CO-Zahl ist ein Maß für den Restaldehydgehalt. Durch den Alkalihydroxidzusatz kann die Bildung von Aldehyden während der destillativen Behandlung vermindert werden und bereits im eingesetzten Alkohol vorhandener Aldehyd beseitigt werden.

Auch US-A-2,533,754 betrifft ein Verfahren zur Reinigung eines aldehydhaltigen Ethanols unter Zusatz von Alkalimetallhydroxiden in einer Menge von 0,1 bis 3 Gew.-%, bezogen auf den zu reinigenden Alkohol. Gemäß einer beispielhaften Ausführung wird einer 88,3 Gew.-%igen Lösung an Ethanol Natriumhydroxid in einer solchen Menge zugesetzt, dass der Natriumhydroxidgehalt in der Lösüng 1,46 Gew.-% beträgt.

Aus US-A-2,889,375 ist ein Verfahren zur destillativen Reinigung von Oxoalkoholen bekannt, die geringe Mengen an Aldehydverbindungen als Verunreinigungen enthalten. Das offenbarte Verfahren ist dadurch charakterisiert, dass dem Kolonnensumpf Erdalkalimetallverbindungen, insbesondere Oxide, Hydroxide oder Carbonate in einer Menge von 1 Gew.-%, bezogen auf den Einsatz, zugesetzt werden. Nach der Lehre von US-A-2,889,375 führt jedoch der Zusatz von Alkaliverbindungen, wie Natriumcarbonat oder Natriumhydroxid während der Destillation zu erheblichen Alkoholverlusten infolge der Bildung von Kondensationsprodukten.

US-A-3,689,371 offenbart ein Verfahren zur destillativen Reinigung von Butanolen aus der Oxosynthese. Der anfallende Rohalkohol wird zunächst mit einer wässrigen Alkalimetallhydroxidlösung behandelt, um die im Rohalkohol enthaltenen Säuren und Ester zu neutralisieren bzw. zu spalten. Nach dem bekannten Verfahren werden zunähst die Alkalihydroxide, beispielsweise durch eine Wasserwäsche, aus dem Rohalkohol entfernt. Anschließend wird der alkalihydroxidfreie Rohalkohol in die weitere destillative Aufarbeitung gefahren.

US 5,227,544 behandelt die Herstellung von 2-Ethylhexanol mit einem verminderten Gehalt an 2-Ethyl-4-methylpentanol. Charakteristisch ist die Destillation eines rohen n-Butyraldehyds, der als Verunreinigungen Oligomere des Isobutyraldehyds enthält, in Gegenwart von Wasser. Durch den Wasserzusatz in der Destillation kommt es zur Spaltung der Oligomeren und freigesetzter Isobutyraldehyd wird als Kopfprodukt entfernt. Der so gereinigte n-Butyraldehyd wird anschließend in Gegenwart von alkalischen Katalysatoren aldolisiert und das Aldolisierungsprodukt wird anschließend zum 2-Ethylhexanol hydriert.

EP 0 216 151 betrifft ebenfalls ein Verfahren zur Herstellung von 2-Ethylhexanol. Hierbei wird Propen in Gegenwart eines Rhodium und sulfonierte Triarylphosphine enthaltenden, in Wasser gelösten Katalysators hydroformyliert und aus dem Reaktionsgemisch werden Isobutyraldehyd und weitere, niedriger als n-Butyraldehyd siedende Bestandteile destillativ abgetrennt. Der anfallende Destillationsrückstand, der im Wesentlichen n-Butyraldehyd enthält, wird in Gegenwart einer wässrigen Alkalilösung aldolisiert und das resultierende Aldolisierungsprodukt zum 2-Ethylhexanol hydriert.

Aus dem Stand der Technik ist bekannt, die Reindestillation der Alkohole in Gegenwart geringer Mengen an Alkalihydroxid durchzuführen, um die Reinheit der destillierten Alkohole zu erhöhen. Der Stand der Technik gibt jedoch keinen Hinweis darauf, die bei der Herstellung und destillativen Aufarbeitung anfallenden Rückstände zu behandeln, um aus den Hochsiedern Alkohole wieder zurückzugewinnen.

Die Erfindung besteht somit in einem Verfahren zur Gewinnung von aliphatischen C₃-C₁₀ Alkoholen aus Hochsiedern, die als Sumpfprodukt bei der Reindestillation der aliphatischen C₃-C₁₀ Alkohole anfallen und die gegebenenfalls mit Hochsiedern aus vorgeschalteten Aldolisierungsprozessen versetzt werden.

Es ist dadurch gekennzeichnet, dass den Hochsiedern eine Alkalimetallverbindung in einer Menge zugesetzt wird, dass die Neutralisationszahl des Hochsieders einen Wert von 2 mgKOH/g nicht unterschreitet, und dass die Hochsieder bei einer Temperatur von 165 bis 185°C und bei einem Druck von 80 bis 150 hPa in einer Destillationskolonne behandelt werden und das abgezogene Kopfabzugsprodukt nachfolgend hydriert wird.

Die in dem erfindungsgemäßen Verfahren eingesetzen Hochsieder fallen als Sumpfprodukt bei der Reindestillation der Alkohole an. Diese Destillationsrückstände werden gegebenenfalls mit weiteren Hochsiedern aus der Herstellung der Alkohole versetzt, bevor sie in einer separaten Destillationskolonne gemäß dem erfindungsgemäßen Verfahren behandelt werden. Derartige Hochsieder fallen beispielsweise bei vorgeschalteten Aldolisierungsprozessen an, in denen Aldehyde zunächst in höherkettige ungesättigte Aldehyde kondensiert werden, die anschließend zu den gesättigten Alkoholen hydriert werden. Die separate Destillationskolonne, in der die Hochsieder gemäß dem erfindungsgemäßen Verfahren behandelt werden, bezeichnet man auch als Rückstandskolonne.

Überraschenderweise führt die erfindungsgemäße Behandlung der Hochsieder aus der Herstellung und Destillation der aliphatischen C₃-C₁₀-Alkohole zu einer Hochsiederspaltung in die entsprechenden Alkohole und Aldehyde, die über den Kolonnenkopf abgezogen werden. Der Kopfabzug der Rückstandskolonne, enthaltend Alkohole und Aldehyde, wird in die Hydrierstufe des Alkoholherstellprozesses zurückgefahren, wobei der Aldehydanteil zu den entsprechenden Alkoholen hydriert wird. Dadurch gelingt es, den Hochsiederanfall um bis etwa 20 % zu reduzieren und die Alkoholausbeute zu erhöhen im Vergleich zu der Arbeitsweise, bei der in einer Rückstandskolonne ohne Alkalizusatz gearbeitet wird.

Bei den über den Sumpf der Reindestillationskolonne ausgeschleusten Hochsiedern und den gegebenenfalls zugesetzten Hochsiedern aus dem Alkoholherstellprozeß handelt es sich um ein komplexes Gemisch, wie beispielsweise Esterverbindungen oder Aldolkondensationsprodukten. Eine verschärfte thermische Belastung in der Reindestillationskolonne führt zwar zu einer Reduzierung des Restalkoholgehalts in den Hochsiedern, fördert aber auch die Hochsiederbildung. Der Restgehalt an aliphatischen C₃-C₁₀ Alkoholen hängt naturgemäß von den Destillationsbedingungen in der Rückstandskolonne ab und liegt im allgemeinen in einem Bereich zwischen 3 bis 5%, bezogen auf die gesamte Rückstandsmenge.

Zur Gewinnung von wertvollen aliphatischen C₃-C₁₀ Alkoholen durch eine gezielte Hochsiederspaltung wird der Hochsiederanfall in einer Rückstandskolonne bei einer Temperatur in einem Bereich von 165 bis 185°C, vorzugsweise von 170 bis 180°C in Gegenwart einer Alkalimetallverbindung behandelt. Die Menge der zugesetzten Alkalimetallverbindung richtet sich nach dem Restsäuregehalt im Hochsiederanfall und sie ist so bemessen, dass die Neutralisationszahl des Hochsieders einen Wert von 2 mgKOH/g nicht unterschreitet. Die Bestimmung der Neutralisationszahl erfolgt gemäß DIN 51 558-01. Vorzugsweise wird die Alkalimetallverbindung in einer solchen Menge zugesetzt, dass sich die Neutralisationszahl im Hochsiederanfall in einem Bereich von 2 bis 5 mgKOH/g bewegt. Bei Spalttemperaturen von unterhalb von 165°C wird keine ausreichende Alkohol- und Aldehydbildung mehr beobachtet, selbst wenn man die Menge der zugesetzten Alkalimetallverbindung erhöht und die Neutralisationszahl auf unter 2 mgKOH/g einstellt. Auch die Erhöhung der Spalttemperatur auf über 185°C führt zu keiner vermehrten Alkohol- und Aldehydbildung mehr, auch selbst dann, wenn der Zusatz an Alkalimetallverbindungen erhöht wird.

Das erfindungsgemäße Verfahren zeichnet sich somit durch die Einhaltung eines engen Temperaturbereichs aus, der in der Rückstandskolonne einzustellen ist, um eine optimale Hochsiederspaltung zu erzielen.

Die durch die Hochsiederspaltung in der Rückstandskolonne gebildeten Alkohole und Aldehyde werden als Kopfprodukt abgezogen und in die Hydrierstufe des Alkoholherstellprozesses zurückgefahren, in der die Aldehyde zu den entsprechenden Alkoholen hydriert werden.

Die Hydierung erfolgt in der Gasphase unter konventionellen Bedingungen in Gegenwart von üblichen Hydrierkatalysatoren, wie z.B. aus EP-B1-0 421 196 oder EP-B1-0 335 222 bekannt. Neben den Trägerkatalysatoren auf Basis von Nickel, Aluminiumoxid und Zirkondioxid können auch Nickel-Katalysatoren, wie z.B. aus EP-B1-0 618 006 bekannt, verwendet werden. Geeignet sind ebenfalls kupferoxidhaltige Katalysatoren, wie z.B. aus EP-A-0 604 792 oder EP-A-0 528 305 bekannt.

Besonders geeignet ist der in der EP-A-0 528 305 beschriebene Hydrierkatalysator, der je 100 Gew.-Teile Kupferoxid, 40-130 Gew.-Teile Zinkoxid, 2-50 Gew.-Teile Aluminiumoxid und gegebenenfalls 0,5-8 Gew.-Teile Mangen-, Molybdän-, Vanadium-, Zirkon- und/oder Erdalkalimetalloxid enthält und eine BET-Gesamtoberfläche von 80-175 m²/g Katalysator im nichtreduzierten Zustand besitzt, wobei 75-95 % der BET-Gesamtoberfläche von Poren eines Radius rₚ ≤ 15 nm gebildet werden.

Die Hydriertemperatur liegt im allgemeinen bei 50-250°C, bevorzugt bei 80-160°C. Der Druck liegt im allgemeinen in einem Bereich von 0,01-2,5 MPa.

Die aliphatischen C₃-C₁₀ Alkohole können geradkettig oder verzweigt sein. Nach dem erfindungsgemäßen Verfahren lassen sich besonders vorteilhaft Hochsieder aus der Herstellung von 2-Ethylhexanol aufarbeiten. Hierbei werden die Destillationsrückstände aus der 2-Ethylhexanol-Reindestillation und die Rückstände, die in der vorgeschalteten Aldolisierung von n-Butyraldehyd zu 2-Ethylhexenal und in der 2-Ethylhexenaldestillation anfallen, vereinigt und nach dem erfindungsgemäßen Verfahren in der Rückstandskolonne behandelt. Das 2-Ethylhexanol, 2-Ethylhexenal und 2-Ethylhexanal enthaltende Kopfprodukt wird in die Hydrierstufe zurückgeführt und anschließend der 2-Ethylhexanolreindestillation zugeführt.

Als Alkalimetallverbindungen werden beispielsweise Hydroxide, Carbonate oder Hydrogencarbonate eingesetzt. Bevorzugt sind Natriumhydroxid oder Kaliumhydroxid. Die Alkalimetallhydroxide werden als wässrige Lösung zugegeben, üblicherweise mit einem Gehalt von 18 bis 25 Gew.-% an Alkalimetallhydroxid, bezogen auf die wässrige Lösung. Ein Zusatz der Alkalimetallverbindungen in fester Form ist jedoch nicht ausgeschlossen.

Die Alkalimetallverbindung wird dem Zulauf der Rückstandskolonne zugesetzt, die sich dann im Sumpf der Rückstandskolonne ansammelt.

Bei der Rückstandskolonne handelt es sich um eine übliche Destillationskolonne, die im allgemeinen von 20 bis 40, vorzugsweise von 25 bis 35 Böden aufweist. Die Rückstandskolonne kann diskontinuierlich oder kontinuierlich betrieben werden.

Durch das erfindungsgemäße Verfahren gelingt es, aus dem Hochsiederanfall aliphatische C₃-C₁₀ Alkohole und Aldehyde zurückzugewinnen, die in einem nachgeschalteten Hydrierprozeß in Alkohole überführt werden. Über den gesamten Alkoholherstellprozess gesehen, wird die Alkoholausbeute erhöht und der gesamte Hochsiederanfall reduziert.

### Beispiel

### A

Die vereinigten Hochsieder aus der 2-Ethylhexanolherstellung wurden durch Zugabe einer wäßrigen Kaliumhydroxidlösung (20%ig) auf eine Neutralisationszahl von 3 mg KOH/g eingestellt und in einer Rückstandskolonne (30 Böden) bei einer Temperatur von 175°C und bei einem Druck von 90 hPa behandelt. Über den Sumpf der Rückstandskolonne wurden pro Stunde 750 kg Hochsieder abgeführt, während der Kopfabzug in die Hydrierstufe zurückgeführt wurde.

### B (Vergleichsbeispiel)

Die Rückstandskolonne wurde wie unter A betrieben, jedoch mit der Ausnahme, dass auf die Zugabe von Kaliumhydroxid verzichtet wurde. Pro Stunde fielen im Sumpf der Rückstandskolonne 930 kg Hochsieder an, die ausgeschleust wurden.

Durch die erfindungsgemäße Zugabe von Alkalimetallverbindungen kann in dem Herstellungsprozeß von 2-Ethylhexanol der Hochsiederanfall um ca. 20% reduziert werden.

## Patentansprüche

1. Verfahren zur Gewinnung von aliphatischen C₃-C₁₀ Alkoholen aus Hochsiedern, die als Sumpfprodukt bei der Reindestillation der aliphatischen C₃-C₁₀ Alkohole anfallen und die gegebenenfalls mit Hochsiedern aus vorgeschalteten Aldolisierungsprozessen versetzt werden, **dadurch gekennzeichnet, dass** den Hochsiedern eine Alkalimetallverbindung in einer Menge zugesetzt wird, dass die Neutralisationszahl des Hochsieders einen Wert von 2 mgKOH/g nicht unterschreitet, und dass die Hochsieder bei einer Temperatur von 165 bis 185°C und bei einem Druck von 80 bis 150 hPa in einer Destillationskolonne behandelt werden und das Kopfabzugsprodukt nachfolgend hydriert wird.

2. Verfahren nach Anspruch 1, **dadurch gekennzeichnet, dass** die Neutralisationszahl durch Alkalimetallzusatz auf einen Bereich von 2 bis 5 mgKOH/g eingestellt wird.

3. Verfahren nach Anspruch 1 oder 2, **dadurch gekennzeichnet, dass** die Temperatur 170 bis 180°C beträgt.

4. Verfahren nach einem oder mehreren der Ansprüche 1 bis 3, **dadurch gekennzeichnet, dass** man eine wässrige Lösung der Alkalimetallverbindung einsetzt.

5. Verfahren nach einem oder mehreren der Ansprüche 1 bis 4, **dadurch gekennzeichnet, dass** die Alkalimetallverbindung ein Alkalimetallhydroxid ist.

6. Verfahren nach Anspruch 5, **dadurch gekennzeichnet, dass** das Alkalimetallhydroxid Natriumhydroxid oder Kaliumhydroxid ist.

7. Verfahren nach einem oder mehren der Ansprüche 1 bis 6, **dadurch gekennzeichnet, dass** man als aliphatischen C₃-C₁₀ Alkohol 2-Ethylhexanol einsetzt.

8. Verfahren nach einem oder mehreren der Ansprüche 1 bis 7, **dadurch gekennzeichnet, dass** die Alkalimetallverbindung dem Zulauf der Destillationskolonne zugesetzt wird.

## Claims

1. A process for producing aliphatic C₃-C₁₀-alcohols from high boilers which are obtained as bottom product in the purification of the aliphatic C₃-C₁₀-alcohols by distillation and which are, if appropriate, admixed with high boilers from upstream aldolization processes, wherein an alkali metal compound is added to the high boilers in an amount such that the neutralization number of the high boiler is not below a value of 2 mg KOH/g, and wherein the high boilers are treated at a temperature of from 165 to 185°C and a pressure of from 80 to 150 hPa in a distillation column and the overhead product taken off is subsequently hydrogenated.

2. The process as claimed in claim 1, wherein the neutralization number is brought to a value in the range from 2 to 5 mg KOH/g by addition of an alkali metal compound.

3. The process as claimed in claim 1 or 2, wherein the temperature is from 170 to 180°C.

4. The process as claimed in one or more of claims 1 to 3, wherein an aqueous solution of the alkali metal compound is used.

5. The process as claimed in one or more of claims 1 to 4, wherein the alkali metal compound is an alkali metal hydroxide.

6. The process as claimed in claim 5, wherein the alkali metal hydroxide is sodium hydroxide or potassium hydroxide.

7. The process as claimed in one or more of claims 1 to 6, wherein the aliphatic C₃-C₁₀-alcohol used is 2-ethylhexanol.

8. The process as claimed in one or more of claims 1 to 7, wherein the alkali metal compound is added to the feed to the distillation column.

## Revendications

1. Procédé pour l'obtention d'alcools aliphatiques en C₃-C₁₀ à partir de composés à haut point d'ébullition qui apparaissent en tant que produit résiduel dans la distillation pour la purification des alcools aliphatiques en C₃-C₁₀ et qui sont éventuellement mélangés avec des composés à haut point d'ébullition provenant de processus aldolisation en amont, **caractérisé en ce qu'**on ajoute aux composés à haut point d'ébullition un composé contenant un métal alcalin, en une quantité telle que l'indice de neutralisation du composé à haut point d'ébullition ne soit pas inférieur à une valeur de 2 mg de KOH/g, et **en ce que** les composés à haut point d'ébullition sont traités à une température de 165 à 185 °C et sous une pression de 80 à 150 hPa dans une colonne de distillation et le produit déchargé à la tête de la colonne est ensuite hydrogéné.

2. Procédé selon la revendication 1, **caractérisé en ce que** l'indice de neutralisation est ajusté dans une plage de 2 à 5 mg de KOH/g par addition d'un métal alcalin.

3. Procédé selon la revendication 1 ou 2, **caractérisé en ce que** la température est de 170 à 180 °C.

4. Procédé selon une ou plusieurs des revendications 1 à 3, **caractérisé en ce qu'**on utilise une solution aqueuse du composé contenant un métal alcalin.

5. Procédé selon une ou plusieurs des revendications 1 à 4, **caractérisé en ce que** le composé contenant un métal alcali est un hydroxyde de métal alcalin.

6. Procédé selon la revendication 5, **caractérisé en ce que** l'hydroxyde de métal alcalin est l'hydroxyde de sodium ou l'hydroxyde de potassium.

7. Procédé selon une ou plusieurs des revendications 1 à 6, **caractérisé en ce qu'**on utilise comme alcool en C₃-C₁₀ le 2-éthylhexanol.

8. Procédé selon une ou plusieurs des revendications 1 à 7, **caractérisé en ce que** le composé contenant un métal alcalin est ajouté au courant d'alimentation de la colonne de distillation.
